(19) Europäisches Patentamt
European Patent Office
Office européen des brevets

(11) Veröffentlichungsnummer: **0 254 949**
**A2**

(12) **EUROPÄISCHE PATENTANMELDUNG**

(21) Anmeldenummer: 87110119.2

(22) Anmeldetag: 14.07.87

(51) Int. Cl.4: **C08G 85/00**, G02F 1/35

(30) Priorität: 23.07.86 DE 3624858

(43) Veröffentlichungstag der Anmeldung: 03.02.88 Patentblatt 88/05

(84) Benannte Vertragsstaaten: BE CH DE FR GB IT LI NL SE

(71) Anmelder: **Merck Patent Gesellschaft mit beschränkter Haftung**
**Frankfurter Strasse 250**
**D-6100 Darmstadt(DE)**

(72) Erfinder: **Eidenschink, Rudolf, Dr.**
**Konrad Adenauer Strasse 1**
**D-6109 Mühltal 1(DE)**
Erfinder: **Dorsch, Dieter, Dr.**
**Reuterallee 24**
**D-6100 Darmstadt(DE)**
Erfinder: **Herz, Claus P., Dr.**
**Berghalde 20**
**D-6900 Heidelberg 1(DE)**

(54) **Orientierte Polymermaterialien.**

(57) Durch ringöffnende Polymerisation cyclischer, polare Gruppen enthaltender Monomere werden senkrecht orientierte Polymermaterialien mit nicht-linearen optischen Eigenschaften erhalten.

EP 0 254 949 A2

Xerox Copy Centre

# Orientierte Polymermaterialien

Die Erfindung betrifft neue Polymermaterialien mit nicht-linearen optischen Eigenschaften sowie Verfahren zu ihrer Herstellung.

Die nicht-lineare Optik beschäftigt sich mit der Wechselwirkung elektromagnetischer Felder in verschiedenen Medien und dem damit verbundenen Entstehen neuer Felder mit veränderten Eigenschaften. Materialien mit nicht-linearen optischen Eigenschaften besitzen eine feldstärkeabhängige dielektrische Suszeptibilität, die eine Reihe dispersiver Prozesse zur Folge hat: die Frequenzverdopplung (second harmonic generation = SHG) erlaubt die Erzeugung von Licht der verglichen mit dem eingestrahlten Licht halben Wellenlänge; der elektrooptische Effekt (Pockels-Effekt) ermöglicht eine Änderung des Brechungsindex bei angelegtem elektrischem Gleichstromfeld; Methoden der Summen-und Differenzfrequenzmischung sowie der Frequenzteilung gestatten die kontinuierliche Abstimmung von Laserlicht.

Eine Vielzahl technischer Anwendungen resultiert aus den voranstehend angeführten Effekten. Optische Schalter und Wellenleiter zur Konstruktion rein-optischer Computer, die Frequenz-und Intensitätssteuerung in der Lasertechnik, Holographie sowie die Bereiche der Informationsverarbeitung und integrierten Optik stellen Einsatzgebiete für Materialien mit nicht-linearen optischen Eigenschaften dar.

Um für die Anwendung im Bereich der nicht-linearen Optik geeignet zu sein, müssen derartige Materialien einer Reihe von Anforderungen genügen.

Neben einer nicht-zentrosymmetrischen Molekülanordnung im Kristall bedingt eine technische Brauchbarkeit möglichst hohe Werte für die dielektrische Suzeptibilität $\chi$.

Eine Reihe anorganischer Substanzen wie z.B. Kaliumdihydrogenphosphat oder Lithiumniobat zeigt nicht-lineare optische Eigenschaften. Alle diese Verbindungen sind jedoch mit den verschiedensten Nachteilen behaftet. Neben unzureichenden Werten für die dielektrische Suszeptibilität zweiter Ordnung mangelt es anorganischen Verbindungen häufig an einer genügenden Photostabilität bei der Behandlung mit hohen Lichtintensitäten oder, bedingt durch eine starke Farbigkeit, an ausreichender Transparenz.

Aus Garito et al., Laser Focus 18 (1982) und der EP-0091 838 sind organische Verbindungen vom Nitroanilintyp bekannt. Ihre verhältnismäßig guten Werte für die photochemische Stabilität und die dielektrische Suszeptibilität zweiter Ordnung gehen jedoch einher mit einer schlechten Kristallisierbarkeit und einer mangelhaften mechanischen Stabilität. Insbesondere die Herstellung dünner Schichten, wie von der integrierten Optik gefordert, gelingt mit diesen Materialien nicht.

Polymere zeichnen sich durch hohe mechanische Widerstandfähigkeit und gute chemische Stabilität aus. Am Polymergerüst befestigte oder in Polymeren gelöste Moleküle mit nicht-linearen optischen Eigenschaften sollten daher in der nicht-zentrosymmetrischen Umgebung vorteilhafte Werte für die dielektrische Suszeptibilität aufweisen.

Polymere mit Nichtlinearitäten zweiter Ordnung lassen sich herstellen, indem an über die Glastemperatur erhitzte, mit statistisch orientierten Molekülen dotierte Filme ein äußeres Feld angelegt wird. Dies führt zu einer Polung der eingelagerten Moleküle, die dem Polymermedium nach dessen Erstarren eine Anisotropie verleiht. Auf diese Weise hergestellte Polymere mit nicht-linearen optischen Eigenschaften, in denen p,p'-Dimethylaminonitrostilben als Wirtsmolekül Verwendung findet, wurden von Meredith et al., Macromolecules 15 - (1982) 1385 beschrieben.

Shibaev et al., Polymer Communications 24 - (1983) 364 teilen die feldinduzierte Ausrichtung flüssigkristalliner Polymerer mit mesogenen Seitengruppen mit.

Die US-Patentschrift 4 412 059 offenbart ein Polymermaterial mit cholesterischen Mesophasen, die mittels elektrischer oder magnetischer Felder einer kontrollierten Ausrichtung zugänglich sind. Schließlich sind aus der EP-0172012 vollaromatische, thermotrope, flüssigkristalline Polymere bekannt, deren nicht-lineare optische Eigenschaften ebenfalls durch äußere Felder hervorgerufen werden können.

Eine weitere Methode zur Erzeugung von Polymermaterialien mit nicht-linearen optischen Eigenschaften besteht in der Polymerisation von bereits geordneten, eine nicht-zentrosymmetrische Orientierung aufweisenden Monomeren, wobei der Ordnungszustand des Systems während der Polymerisation weitgehend erhalten bleibt. Für diese Technik geeignete Monomere sind beispielsweise der EP-0021695 zu entnehmen.

Die nach den voranstehend beschriebenen Verfahren erhaltenen Materialien zeigen noch unbefriedigende nicht-lineare optische Eigenschaften. Durch die Einwirkung eines äußeren Feldes, welches einen zusätzlichen Verfahrensschritt darstellt, oder durch Orientierungsverluste der vorgeordneten Monomeren während der Polymerisation erfolgt nur eine unvollkommene Ausrichtung des Polymerverbandes.

Es besteht somit ein Bedürfnis nach auf eine Oberfläche aufgebrachten, orientierten Polymermaterialien sowie nach einem Verfahren zu deren Herstellung, das die geschilderten Nachteile nicht oder nur in geringerem Maße aufweist und das insbesondere ohne die Erfordernis zusätzlicher Verfahrenschritte eine Orientierung der Polymerenstruktur bereits während des Polymeristationsvorganges ermöglicht.

Diese Aufgabe wird gelöst durch die erfindungsgemäßen Polymermaterialien sowie durch Verfahren zu deren Herstellung.

Gegenstand der Erfindung sind daher auf eine Oberfläche aufgebrachte, aus polaren Monomereinheiten bestehende Polymermaterialien, dadurch gekennzeichnet, daß die Monomereinheiten und die Polymerhauptketten eine zur Substratoberfläche vorzugsweise senkrechte Orientierung aufweisen. Bei einer besonders bevorzugten Gruppe der erfindungsgemäßen Polymermaterialien sind die Monomereinheiten vorzugsweise dipolar ausgerichtet. Der Grad der Senkrechtorientierung beträgt vorzugsweise mindestens etwa 60 % bei einer Winkelabweichung von der Senkrechten von höchstens etwa 10°, insbesondere etwa 80% bei einer Winkelabweichung von etwa 5°.

Gegenstand der Erfindung ist ferner eine Verfahren zur Herstellung derartiger Polymermaterialien, indem man zu ringöffnender Polymerisation befähigte, polare Gruppen enthaltende, cyclische Monomere in Gegenwart eines auf der Substratoberfläche verankerten Polymerisationsinitiators polymerisiert, oder indem man polare Gruppen enthaltende Monomere polymerisiert und anschließend auf einer Substratoberfläche verankert.

Nach diesen Verfahren erhaltene Polymermaterialien eignen sich als optisch nicht-lineare Medien.

Die ringöffnende Polymerisation cyclischer Monomerer ist bekannt. So werden in Frisch, Reegen (Hrsg.), Ring-opening Polymerization, Marcel Dekker, New York, 1969, Saegusa, Goethals (Hrsg.), Radical Polymerization, American Chemical Society, Washington, 1977 sowie Yvin, Saegusa (Hrsg.), Ring-Opening Polymerization, Elsevier, London, 1984, Verfahren zur ionischen Ringöffnung und nachfolgenden Polymerisation von beispielsweise Ethylenoxid, Tetrahydrofuran, Caprolacton und Caprolactam beschrieben.

Radikalischer Ringöffnung und Polymerisation zugängliche Monomere sind z.B. Bicyclobutane (Hall, Ykman, J. Macromol. Sci, Rev. Macromol. Chem. 11 (1976)), Olefine (Errede, J. Polym. Sci., 49 (1961) 253), Spiroorthocarbonate (Endo, Bailey, J. Polym. Sci., Polym. Lett. Ed. 18 (1980) 25) oder cyclische Ketenacetale (Endo et al., Makromol.Chem. 186 (1985) 1543); wobei die Radikalbildung auf thermischem oder photochemischem Weg erfolgen kann.

Es wurde nun überraschenderweise gefunden, daß durch ringöffnende Polymerisation polare Gruppen enthaltender, cyclischer Monomeren der allgemeinen Formel I,

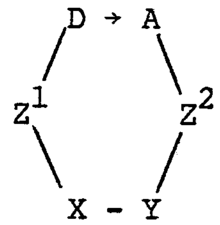

I

worin D → A ein polare Gruppen enthaltendes Molekülfragment, $Z^1$ und $Z^2$ eine unsubstiuierte oder substituierte Kohlenstoffkette, in der gegebenenfalls ein oder mehrere C-Atome durch Heteroatome ersetzt sind, und X - Y eine unter Erzeugung polymerisationsfähiger Fragmente spaltbare Gruppe bedeuten,

in Gegenwart eines auf der Substratoberfläche verankerten Polymerisationsinitiators neuartige Polymermaterialen erhalten werden, deren Monomereinheiten und Polymerhauptketten zur Substratoberfläche vorzugsweise senkrechte Orientierung aufweisen. Derartige Polymermaterialien eignen sich in hervorragender Weise als nicht-lineare optische Medien.

Die Ausführung der Reaktion gestaltet sich einfach. In den zu polymerisierenden cyclischen Monomeren der Formel I besitzt in dem polare Gruppen enthaltenden Molekülfragment D → A D die Eigenschaften einer Elektronendonorgruppe, wie z.B. eine Amino-, Ether-, Phosphin-oder Alkoholgruppe, A die Eigenschaften einer Elektronenakzeptorgruppe, wie z.B. eine Nitro-, Cyano-, Ester- oder Anhydridgruppe, ein Chinon oder Halogen. Vorzugsweise bedeutet A eine Amino-oder Alkoholgruppe, B eine Nitro-oder Estergruppe.

Vorgenannte Gruppen befinden sich an einem organischen Gerüst, vorzugsweise einem aromatischen System. Hierfür geeignete Systeme sind beispielsweise in 1,2-oder 1,4-Stellung durch die Gruppen D und A substituiertes Benzol, in 4,4'-Stellung substituiertes Biphenyl oder Stilben oder in 2,6-Stellung substituiertes Naphthalin.

$Z^1$ und $Z^2$ repräsentieren eine Alkylkette mit 1 bis 18 C-Atomen, in der auch eine oder mehrere $CH_2$-Gruppen durch -O-, -CO-, -CO-O-, -NH-, -N-(Alkyl)-oder -CH=CH-substituiert sein können. -O-, -CO-O-und -NH-sind als ersetzende Gruppen bevorzugt.

Die unter Erzeugung polymerisationsfähiger Fragmente spaltbare Gruppe X-Y bezeichnet ein radikalisch, ionisch oder thermisch spaltbares Strukturelement, wie z.B. Peroxide (X-Y = O-O),

cyclische Orthoester (O-CHR-O; R = organischer Rest), cyclische Orthocarbonate (O-C(OR)$_2$-O) oder Ketenacetale (O-C(= CR$_2$)-O), wobei letztere eine bevorzugte Gruppe X-Y darstellen.

Cyclische Monomere mit diesen reaktiven Gruppen sind nach den aus den voranstehend angegebenen Schriften entnehmbaren Vorschriften oder nach an sich bekannten Methoden erhältlich, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

So sind beispielsweise die besonders bevorzugten Monomeren der Formel I, in den X-Y eine Ketenacetalfunktion bedeutet, aus 2-Halogenmethyl-1,3-dioxacycloverbindungen durch Einwirkung einer starken Base wie z.B. Natriumamid oder Kalium-tert.-butanolat in einem inerten Lösungsmittel erhältlich.

Die zur Auslösung der Polymerisation erforderlichen Photoinitiatoren werden in geeigneter Weise auf dem Substrat verankert. Eine Haftung auf dem jeweiligen Untergrund kann aufgrund physikalischer, beispielsweise adsorptiver, oder chemischer Wechselbeziehungen erreicht werden. Physikalisch haftende Photoinitiaren können beispielsweise durch Bestreichen, Bedrucken, Bedampfen oder Tauchen auf das Substrat aufgebracht werden. Nach Entfernen überschüssigen, nicht haftenden Photoinitiators können die so beschichteten Substrate zur Verwendung im erfindungsgemäßen Verfahren direkt mit einem polymerisierbaren Monomerenüberzug versehen werden.

Zur Auslösung der Polymerisation geeignet ist eine Vielzahl bekannter Initiatoren, wie z.B. in Pappas (Hrsg.), UV Curing: Science and Technology, Technology Marketing Corp., Stamford, CT, 1978, oder in Ocian, Principles of Polymerization, McGraw-Hill, New York, beschrieben. Beispiele für thermisch zerfallende, radikalische Initiatoren sind Perverbindungen wie Kaliumpersulfat, Dibenzolylperoxid und Cyclohexanonperoxid, für durch Strahlungseinwirkung zerfallende Initiatoren Benzophenone wie z.B. Michlers Keton [4,4'-Bis-(dimethylamino)benzophenon], 4,4'-Bis-(diäthylamino)benzophenon, p-Dimethylaminobenzophenon, p-Chlorbenzophenon, Benzophenon; Anthrachinone wie z.B. Anthrachinon, 2-Chloranthrachinon, 2-Alkylanthrachinone; Xanthone wie z.B. 2-Halogenxanthone, 2-Alkyl-xanthone; Thioxanthone wie 2-Chlorthioxanthon, 2-Alkylthioxanthone; Acridanone wie z.B. 2-Alkylacridanone oder N-substituierte Acridanone; Benzoine wie z.B. p-Dimethylaminobenzoin und Alkylether des Benzoins; Benzilketale, α-Halogenketone, Dialkoxyacetophenone, α-Hydroxyalkylphenone und α-Aminoalkylphenone wie sie beispielsweise in der DE-OS 27 22 264 und in der EP-OS 3003 beschrieben sind, weiterhin z.B. Fluorenone, Dibenzosuberone, Phenanthrenchinone, Benzoesäureester wie z.B. Hydroxypropylbenzoat, Benzolybenzoatacrylat sowie Oniumsalze wie z.B. Diaryliodonium-oder Triarylsulfoniumsalze.

Ionische Katalysatoren sind z.B. Hydroxide wie Kaliumhydroxid, alkali-organische Verbundungen wie Phenyllithium und Naphthalinnatrium, Lewissäuren wie $BF_3$, $AlCl_3$, $SnCl_4$ und $TiCl_4$, Metallkomplexe in Form von Aluminium-oder Titanverbindungen und starke Säuren wie Fluorsulfonsäure.

Chemisch auf der Substratoberfläche haftende Photinitiatoren sind über eine chemische Bindung an reaktive Gruppen des Substratmaterials gebunden.

Eine besonders bevorzugte Methode zur Fixierung von Photoiniatoren auf hydroxy-funktionalisierten Oberfläche besteht in deren Umsetzung mit difunktionellen Silanen der allgemeinen Formel II,

$$X_3Si\text{-}Z\text{-}Ar\text{-}CO\text{-}C(OR^1)R^2R^3 \qquad II$$

worin Ar einen unsubstituierten oder substituierten Arylrest, $R^1$ H, Alkyl oder Alkanoyl, $R^2$ und $R^3$ H, Alkyl oder Aryl, einer der Reste $R^2$, $R^3$ auch $OR^1$, X eine zur Reaktion mit der Substratoberfläche befähigte funktionelle Gruppe und Z eine Alkylengruppe bedeutet, in der eine oder mehrere $CH_2$-Gruppen durch Heteroatome ersetzt sein können.

Vorzugsweise bedeutet Ar 1,4-Phenylen, $R^1$ H oder, falls einer der Reste $R^2$ oder $R^3$ $OR^1$, auch Alkyl, $R^2$ und $R^3$ Alkyl oder zusammen Alkylen und X Halogen, Alkoxy oder Alkanoyloxy.

Zur Herstellung der reaktive Silangruppen enthaltenden Photoinitiatoren kann man von Methoden, wie sie beispielsweise aus Deschler et al., Angew. Chem 98 (1986) 237 oder aus der DE-35 21 201 bekannt sind, Gebrauch machen. Dabei können auch an sich bekannte, hier jedoch nicht näher erwähnte Varianten zur Anwendung gelangen. Die Aufbringung der funktionalisierten Photoinitiatoren erfolgt zweckmäßigerweise mittels Streich-, Druck-oder Tauchtechniken. Eine thermische Nachbehandlung, gegebenenfalls bei gleichzeitiger Beaufschlagung mit einer Wasserdampfatmosphäre, kann zur Vervollständigung des Umsatzes und/oder der Verkürzung der Reaktionsdauer bisweilen angezeigt sein.

Auf die mit einer Initiatorschicht versehenen Substratoberflächen werden die zu polymerisierenden cyclischen Monomeren in der gewünschten Schichtdicke in der voranstehend beschriebenen Weise aufgebracht und durch Zufuhr thermischer Energie oder durch Bestrahlen polymerisiert.

Die thermische Polymerisation gelingt beispielsweise durch einfaches Erhitzen, durch Behandeln mittels Ultraschall oder Mikrowellen oder durch Einwirkung von IR-Strahlung.

Die Photopolymerisation erfolgt nach an sich bekannten Methoden durch Bestrahlen mit Licht oder UV-Strahlung des Wellenlängenbereichs von 250 bis 500 nm, vorzugsweise von 300 bis 400 nm. Als Strahlenquellen könne Sonnenlicht oder künstliche Strahler verwendet werden. Vorteilhaft sind z.B. Quecksilberdampf-Hochdruck-, -Mitteldruck-oder Niederdrucklampen, Xenon-und Wolframlampen; Laser-Lichtquellen können ebenfalls eingesetzt werden.

In einem weiteren Verfahren zur Herstellung der erfindungsgemäßen, senkrecht orientierten Polymermaterialien werden polare Gruppen enthaltende Monomer polymerisiert und anschließend auf einer Substratoberfläche verankert.

Geeignete Monomere werden durch gebräuchliche Methoden der Polymerisation, Polykondensation und/oder Polyaddition zu polaren Polymeren der allgemeinen Formel III,

$$H\text{-}(Z^1\text{-}D\rightarrow A\text{-}Z^2\text{-})_nV \quad \text{III}$$

worin D→A und $Z^1$ und $Z^2$ die für Formel I angegebene Bedeutung besitzen, n eine den Polymerisationsgrad ausdrückende ganze Zahl und V eine funktionelle, gegebenenfalls nach Modifizierung, zur Verankerung auf das Substrat geeignete Gruppe bedeuten,
umgesetzt.

Vorzugsweise haben D→A und $Z^1$ und $Z^2$ die für Formel I angegebene Bedeutung; n ist vorzugsweise 10-1000, insbesondere 20-100, V bedeutet bevorzugt $-NH_2$, -OH oder -SH, insbesondere -OH.

Eine besonders bevorzugte Gruppe von Polymeren der Formel III liegt in den Polyestern vor. Methoden zu ihrer Herstellung sind beispielsweise aus Jin et al., Br. Polym. J. 12 (1980) 132 und Preston, Ang. Makromol. Chem. 109/110 (1982) 1, zu entnehmen.

Die Funktionalisierung der Polyester mittels reaktiver Silane kann nach Deschler, loc. cit., oder nach anderen, aus dem Schrifttum bekannten, beispielsweise in Houben-Weyl, Methoden oder Organischen Chemie, Georg-Thieme-Verlag, Stuttgart, Bd. 13/5, aufgeführten Methoden erfolgen.

Beim Aufbringen dieser siliciumfunktionalisierten Polyester auf geeignete Substrate durch beispielsweise Streich-, Tauch-oder Drucktechniken erfolgt eine chemische Bindung zur Substratoberfläche. Nach Entfernen von überschüssigem, nicht umgesetztem Polyester wird eine auf der Oberfläche fest haftende Polymerschicht erhalten, deren polare Monomereinheiten und Polymerhauptketten vorzugsweise senkrecht orientiert sind.

Durch Änderung des Polymerisationsgrades der eingesetzten Polymeren läßt sich die Dicke der Beschichtung in weiten Grenzen variieren und damit dem jeweiligen Anwendungszweck anpassen.

Solcherart hergestellte Polymermaterialien zeigen die nämlichen vorteilhaften nicht-linearen optischen Eigenschaften, wie jene voranstehend beschriebenen, durch Polymerisation an polare Gruppen enthaltenden, cyclischen Monomeren in Gegenwart eines auf der Substratoberfläche verankerten Polymerisationsinitiators erhaltenen Materialien.

Die erfindungsgemäßen Polymermaterialien erschließen aufgrund ihrer vorteilhaften nicht-linearen optischen Eigenschaften eine weites Anwendungsfeld. Insbesondere eignen sie sich zur Frequenzverdoppelung von Laserlicht sowie zur Herstellung von Schaltelementen, Wellenleitern und Phasenmodulatoren auf dem Gebiet der integrierten Optik.

Zur Erläuterung der Erfindung dienen folgende Beispiele:

## Beispiel 1

### Herstellung der Ausgangsmaterialien

a) Eine Lösung von 26,6 g 4'-(6-Hydroxyhexyloxy)-(1-1')-biphenyl-4-carbonsäure, 16 g Epoxystyrol und 16 g Triethylamin in 300 ml Tetrahydrofuran wird 140 Std. zum Sieden erhitzt. Man verdünnt mit 1000 ml Dichlormethan, wäscht zweimal mit 2 N Salzsäure und trocknet über Natriumsulfat. Nach Einengen unter vermindertem Druck wird der Rückstand aus Ethanol kristallisiert. Man erhält 2-Hydroxy-2-phenylethyl-(4'-(6-hydroxyhexyloxy)-(1-1')-biphenyl-4-carboxylat); F: 148-150°.

b) Ein Gemisch aus 6,1 g 2-Hydroxy-2-phenylethyl-(4'-(6-hydroxyhexyloxy)-(1-1')-biphenylcarboxylat), 4,4 g Chloracetaldehyddimethylacetal und 3 mg p-Toluolsulfonsäure wird 2 Tage auf einer Temperatur von 140° gehalten. Während der Reaktion gebildetes Methanol wird dabei kontinuierlich abdestilliert. Nach Trocknen unter vermindertem Druck erhält man das cyclische Acetal 9-Chlormethyl-14-oxo-11-phenyl-1,8,10,13-tetraoxa-[14](4,4')-biphenylophan als zähes Öl.

c) Eine Lösung von 1,98 g 9-Chlormethyl-14-oxo-11-phenyl-1,8,10,13-tetraoxa[14](4,4')-biphenylophan, 532 mg Kalium-tert. butanolat und 8 ml tert. Butanol wird 24 Std. auf 80° erhizt. Nach Zugabe von 12 ml Petrolether trennt man das ausgefallene Kaliumchlorid über ein Membranfilter ab. Das Filtrat wird eingeengt und unter vermindertem Druck getrocknet. Es hinterbleibt 9-Methylen-14-oxo-11-phenyl-1,8,10,13-tetraoxa[14](4,4')-biphenylophan als zähes, farbloses Öl.

d) Zu der Lösung von 5,05 g Allylbenzoin und 6.57 g Triethoxysilan fügt 0,1 ml einer 0,02 M Lösung von Tetrachlorplatin(II)-säure-hexahydrat in 2-Propanol und erhitzt anschließend 70 Std. zum Sieden. Es wird eingeengt und 16 Std. unter vermindertem Druck getrocknet, worauf α-(3-Triethoxysilyl-propyl)-benzoin als farbloses Öl hinterbleibt.

Analog werden erhalten:

2-Hydroxy-2-methyl-1-(4-(3-triethoxysilyl-propyloxy)-phenyl)-propan-1-on
2-Hydroxy-2-methyl-1-(4-(2-(3-triethoxysilyl-propyloxy)-ethoxy)-phenyl)-propan-1-on

Beispiel 2

Eine 1%ige Lösung von α-(3-Triethoxysilyl-propyl)-benzoin in Ethanol wird durch Tauchen auf ein Glassubstrat aufgebracht. Hernach wird 10 Min. bei 180° getrocknet und anschließend die Oberfläche mit Isopropanol gespült.

Das mit Initiator belegte Glassubstrat wird durch Tauchen in eine 10%ige Lösung des cyclischen Ketenacetals 9-Methylen-14-oxo-11-phenyl-1,8,10,13-tetraoxa[14](4,4')-biphenylophan aus Beispiel 1c mit einer ca. 3 µm dicken Schicht dieses Monomeren versehen. Zur Polymerisation bestrahlt man 5 Min. mit einer Quecksilberdampflampe. Nach Entfernen von unumgesetzten Monomeren durch Spülen mit Tetrahydrofuran erhält man eine farblose, festhaftende Polymerschicht von hohem Senkrechtorientierungsgrad der Monomereinheiten und Polymerhauptketten, welche sehr gutes Frequenzverdopplungsvermögen aufweist.

Beispiel 3

a) Ein Gemisch aus 629 mg 4'-(6-Hydroxy-hexyloxy)-(1,1')-biphenyl-4-carbonsäure, 144 mg 6-(6-Hydroxyhexyloxy)-naphthalin-2-carbonsäure und 4 mg p-Toluolsulfonsäure wird 4 Std. unter einem Druck von 10 Torr auf 125°, anschließend 1 Std. unter einem Druck von 0,1 Torr auf 230° erhitzt. Das entstandene Polymer ist von glasartiger Beschaffenheit und zeigt nematisch-flüssigkristalline Eigenschaften (Klärpunkt 200°) bei einem durchschnittlichen Molekulargewicht von ca. 14 000.

Analog erhält man aus 4'-(6-Hydroxyhexyloxy)-(1,1')-biphenyl-4-carbonsäure das entsprechende Hompolymer; kristallin, Schmelzpunkt 235°.

b) 140 mg des auf voranstehend angegebene Weise erhältlichen Copolymers werden mit 50 µl Octanol versetzt und 3 h auf 210° erhitzt und anschließend bei der selben Temperatur unter einem Druck von 10 Torr von flüchtigen Anteilen befreit.

Man löst den Rückstand in 3 ml N-Methylpyrrolidon und fügt 150 µl Tetrachlorsilan zu. Hernach wird das Reaktionsgefäß kurzfristig mit einem Druck von 10 Torr beaufschlagt.

Die erhaltene Lösung des silanfunktionalisierten Polyesters bringt man durch Tauchen auf ein Glassubstrat auf. Nach 20 Min. Erwärmen auf 210° hat sich ein festhaftender Polymerfilm gebildet, dessen Polymerhauptketten und Monomereinheiten zur Substratoberfläche in hohem Maße senkrecht orientiert sind und der ausgeprägte, nicht-lineare optische Eigenschaften besitzt.

**Ansprüche**

1. Auf eine Oberfläche aufgebrachte, aus polaren Monomereinheiten bestehende Polymermaterialien, dadurch gekennzeichnet, daß die Monomereinheiten und die Polymerhauptketten eine zur Substratoberfläche vorzugsweise senkrechte Orientierung aufweisen.

2. Polymermaterialien gemäß Patentanspruch 1, dadurch gekennzeichnet, daß die Monomereinheiten eine vorzugsweise dipolare Ausrichtung aufweisen.

3. Verfahren zur Herstellung von Polymermaterialien gemäß Patentanspruch 1, indem man zu ringöffnender Polymerisation befähigte, polare Gruppen enthaltende, cyclische Monomere in Gegenwart eines auf der Substratoberfläche verankerten Polymerisationsinitiators polymerisiert.

4. Verfahren zur Herstellung von Polymermaterialien gemäß Patentanspruch 1, indem man polare Gruppen enthaltende Monomere polymerisiert und anschließend auf einer Substratoberfläche verankert.

5. Verwendung von Polymermaterialien gemäß Patentanspruch 1 als nicht-lineare optische Medien.